(19) Europäisches Patentamt
European Patent Office
Office européen des brevets

(11) **EP 2 828 510 B1**

(12) **EUROPÄISCHE PATENTSCHRIFT**

(45) Veröffentlichungstag und Bekanntmachung des Hinweises auf die Patenterteilung:
**13.01.2016 Patentblatt 2016/02**

(21) Anmeldenummer: **13701627.5**

(22) Anmeldetag: **28.01.2013**

(51) Int Cl.:
*F02D 41/14* *(2006.01)*  *F02D 41/22* *(2006.01)*

(86) Internationale Anmeldenummer:
**PCT/EP2013/051553**

(87) Internationale Veröffentlichungsnummer:
**WO 2013/139512 (26.09.2013 Gazette 2013/39)**

(54) **VERFAHREN UND VORRICHTUNG ZUR ÜBERWACHUNG VON GAS-SENSOREN**

METHOD AND DEVICE FOR MONITORING GAS SENSORS

PROCÉDÉ ET DISPOSITIF POUR LA SURVEILLANCE DE CAPTEURS DE GAZ

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **20.03.2012 DE 102012204353**

(43) Veröffentlichungstag der Anmeldung:
**28.01.2015 Patentblatt 2015/05**

(73) Patentinhaber: **Robert Bosch GmbH**
**70442 Stuttgart (DE)**

(72) Erfinder: **MICHALSKE, Andreas**
**70806 Kornwestheim (DE)**

(56) Entgegenhaltungen:
**DE-A1-102008 001 121      DE-A1-102008 042 549**
**DE-A1-102009 028 367**

**Beschreibung**

Stand der Technik

Die Erfindung betrifft ein Verfahren zur Überwachung von Gas-Sensoren einer Brennkraftmaschine, wobei die Gas-Sensoren abhängig von Geometrie, Messprinzip, Alterung oder Verschmutzung ein Tiefpassverhalten aufweisen, wobei bei der zu messenden Gaszustandsgröße ein Vergleich eines modellierten und eines gemessenen Signals eine Diagnose durchgeführt wird und wobei das gemessene Signal ein Istwert eines Ausgangssignals des Gas-Sensors und das modellierte Signal ein Modellwert ist.

Die Erfindung betrifft weiterhin eine Vorrichtung zur Durchführung des Verfahrens.

Zur Reduktion der Emissionen in PKW mit Ottomotoren werden üblicherweise 3-Wege-Katalysatoren als Abgasreinigungsanlagen verwendet, die nur dann ausreichend Abgase konvertieren, wenn das Luft-Kraftstoffverhältnis $\lambda$ mit hoher Präzision eingeregelt wird. Zu diesem Zweck wird das Luft-Kraftstoffverhältnis $\lambda$ mittels einer der Abgasreinigungsanlage vorgelagerten Abgassonde gemessen. Das Speichervermögen einer derartigen Abgasreinigungsanlage für Sauerstoff wird dazu ausgenutzt, in Magerphasen Sauerstoff aufzunehmen und in Fettphasen wieder abzugeben. Hierdurch wird erreicht, dass oxidierbare Schadgaskomponenten des Abgases konvertiert werden können. Eine der Abgasreinigungsanlage nachgeschaltete Abgassonde dient dabei der Überwachung der Sauerstoff-Speicherfähigkeit der Abgasreinigungsanlage. Die Sauerstoff-Speicherfähigkeit muss im Rahmen der On-Board-Diagnose (OBD) überwacht werden, da sie ein Maß für die Konvertierungsfähigkeit der Abgasreinigungsanlage darstellt. Zur Bestimmung der Sauerstoff-Speicherfähigkeit wird entweder die Abgasreinigungsanlage zunächst in einer Magerphase mit Sauerstoff belegt und anschließend in einer Fettphase mit einem im Abgas bekannten Lambdawert unter Berücksichtigung der durchtretenden Abgasmenge entleert oder die Abgasreinigungsanlage zunächst in einer Fettphase von Sauerstoff entleert und anschließend in einer Magerphase mit einem im Abgas bekannten Lambdawert unter Berücksichtigung der durchtretenden Abgasmenge aufgefüllt. Die Magerphase wird beendet, wenn die der Abgasreinigungsanlage nachgeschaltete Abgassonde den Sauerstoff detektiert, der nicht mehr von der Abgasreinigungsanlage gespeichert werden kann. Ebenso wird eine Fettphase beendet, wenn die Abgassonde den Durchtritt von fettem Abgas detektiert. Die Sauerstoff-Speicherfähigkeit der Abgasreinigungsanlage entspricht der während der Fettphase zur Entleerung zugeführten Menge an Reduktionsmittel bzw. während der Magerphase zur Auffüllung zugeführten Menge an Sauerstoff. Die genauen Mengen werden aus dem Signal der vorgelagerten Abgassonde und dem aus anderen Sensorsignalen ermittelten Abgasmassenstrom berechnet.

Nimmt die Dynamik der vorgelagerten Abgassonde ab, z.B. auf Grund von Verschmutzungen oder Alterung, so kann das Luft-Kraftstoff-Verhältnis nicht mehr mit der erforderlichen Präzision eingeregelt werden, so dass die Konvertierungsleistung der Abgasreinigungsanlage nachlässt. Weiterhin können sich Abweichungen in der Diagnose der Abgasreinigungsanlage ergeben, die dazu führen können, dass eine an sich korrekt arbeitende Abgasreinigungsanlage fälschlich als nicht funktionsfähig bewertet wird. Der Gesetzgeber verlangt eine Diagnose der Sondeneigenschaften während des Fahrbetriebs, um sicherzustellen, dass das geforderte Luft-Kraftstoffverhältnis weiterhin ausreichend genau eingestellt werden kann, die Emissionen die zulässigen Grenzwerte nicht überschreiten und die Abgasreinigungsanlage korrekt überwacht wird. Die OBDII-Bestimmungen verlangen, dass Lambdasonden und andere Abgassonden nicht nur bezüglich ihrer elektrischen Funktionstüchtigkeit überwacht werden, sondern auch hinsichtlich ihres Ansprechverhaltens, d.h. es muss eine Verschlechterung der Sonden-Dynamik erkannt werden, die sich durch eine vergrößerte Zeitkonstante und/ oder Totzeit bemerkbar machen kann. Tot- und Verzögerungszeiten zwischen einer Veränderung der Abgaszusammensetzung und deren Erkennung müssen on-board darauf geprüft werden, ob sie für die Anwenderfunktionen, d.h. für Steuer-, Regel- und Überwachungsfunktionen, die das Sondensignal verwenden, noch zulässig sind. Als Kenngrößen für die Dynamikeigenschaften von Abgassensoren werden typischerweise die Totzeit von einer Gemischänderung bis zur Signalflanke und eine bestimmte Anstiegszeit, z.B. von 0 % auf 63 % oder von 30 % auf 60 % eines Signalhubs, verwendet. Die Totzeit enthält auch die Gaslaufzeit vom Motorauslass bis zur Sonde und verändert sich demnach insbesondere bei einer Manipulation des Sensoreinbauortes.

Bei Dieselmotoren werden als Gas-Sensoren oder Gaskonzentrationssensoren Breitband-Lambdasonden und, in Verbindung mit SCR-Katalysatoren, auch $NO_x$-Sensoren eingesetzt. Letztere liefern zusätzlich ebenfalls ein $O_2$-Signal. Das $O_2$-Signal von Breitband-Lambdasonde oder $NO_x$-Sensor wird beim Dieselmotor nicht nur für den Betrieb von Abgasnachbehandlungseinrichtungen verwendet, sondern auch für die innermotorische Emissionsreduktion. Die gemessene $O_2$-Konzentration im Abgas bzw. das gemessene Lambdasignal wird genutzt, um das Luft-Kraftstoffgemisch dynamisch genau einzustellen und so die Streuungen der Rohemissionen zu minimieren. Bei Dieselmotoren mit $NO_x$-Speicherkatalysator (NSC) wird je eine Breitband-Lambdasonde vor und nach Katalysator für eine zuverlässige Darstellung des Fettbetriebs zur Regeneration benötigt. Innermotorische Emissionsreduktion und NSC-Betrieb stellen ebenfalls bestimmte Mindestanforderungen an die Dynamikeigenschaften der $O_2$-Sonde. Die Anstiegszeit des $O_2$-Signals wird heutzutage beim Übergang von Last nach Schub überwacht, d.h. beim Anstieg von einem bestimmten Prozentsatz unter dem normalen $O_2$-Gehalt von Luft auf 21 %. Erreicht das Sensorsignal nach einer Maximalzeit nicht einmal einen bestimmten Zwischenwert, wird dies als Totzeitfehler interpretiert. Bei Dieselmotoren mit $NO_x$-Speicher-katalysator (NSC) wird daneben üblicherweise das Ansprechverhalten der Lambdasonden vor und nach Katalysator verglichen.

Für kommende Fahrzeuggenerationen bzw. Modelljahre ist zu erwarten, dass auch eine Überwachung der Sensordynamik bei fallender $O_2$-Konzentration gefordert wird. Außerdem wird es bei Hybridfahrzeugen künftig keine Schubphasen mehr geben und somit keine Phasen mit einer konstanten $O_2$-Konzentration von 21 %. Erste Lösungsansätze für diese Zusatzanforderungen sind die aktive Überwachung in der DE 10 2008 001 121 A1 sowie die beobachterbasierte Methode in der DE 10 2008 040 737 A1.

Aus der DE 10 2008 040 737 A1 ist ein Verfahren zur Überwachung von dynamischen Eigenschaften einer Breitband-Lambdasonde bekannt, wobei mittels der Breitband-Lambdasonde ein gemessenes Lambdasignal bestimmt wird, das einer SauerstoffKonzentration im Abgas einer Brennkraftmaschine entspricht, wobei der Brennkraftmaschine ein Beobachter zugeordnet ist, der aus Eingangsgrößen ein modelliertes Lambdasignal erzeugt und wobei aus der Differenz des modellierten Lambdasignals und des gemessenen Lambdasignals oder aus der Differenz aus einem aus dem modellierten Lambdasignal abgeleiteten Signal und einem aus dem gemessenen Lambdasignal abgeleiteten Signal ein Schätzfehler-Signal als Eingangsgröße eines in dem Beobachter einem Modell vorgeschalteten Reglers gebildet wird. Dabei ist vorgesehen, dass ein Maß für die durch eine Totzeit und eine Reaktionszeit charakterisierten dynamischen Eigenschaften der Breitband-Lambdasonde aus einer Bewertung des Schätzfehler-Signals oder einer daraus abgeleiteten Größe bestimmt wird und dass das Maß für die dynamischen Eigenschaften mit vorgegebenen Grenzwerten verglichen wird um zu bewerten, inwiefern die dynamischen Eigenschaften der Breitband-Lambdasonde für einen vorgesehenen Betrieb der Brennkraftmaschine ausreichen.

In der DE 10 2008 001 569 A1 wird zudem ein Verfahren und eine Vorrichtung zur Online-Adaption eines LSU-Dynamikmodells beschrieben. Konkret betrifft die Schrift ein Verfahren und eine Vorrichtung zur Adaption eines Dynamikmodells einer Abgassonde, die Bestandteil eines Abgaskanals einer Brennkraftmaschine ist und mit der ein Lambdawert zur Regelung einer Luft-Kraftstoff-Zusammensetzung bestimmt wird, wobei in einer Steuereinrichtung bzw. in einer Diagnoseeinrichtung der Brennkraftmaschine parallel dazu ein simulierter Lambdawert berechnet wird und von einer Anwender-funktion sowohl der simulierte als auch der gemessene Lambdawert verwendet wird. Dabei ist vorgesehen, dass im laufenden Fahrzeugbetrieb durch Auswerten einer Signaländerung bei Anregung des Systems ein Sprungverhalten der Abgassonde bestimmt und anhand dieser Ergebnisse das Dynamikmodell der Abgassonde adaptiert wird.

Für die Identifikation der Sensoreigenschaften wird dabei auf bekannte Funktionen zur Dynamiküberwachung von Breitband-Lambdasonden zurückgegriffen. Für andere Gaskonzentrationssignale von Abgassensoren, z.B. für ein $NO_x$-Signal, gelten vergleichbare Anforderungen wie für $O_2$-Signale bzw. -Sensoren. Ähnlichkeiten zwischen den Überwachungsfunktionen sind daher anzunehmen.

Das Verfahren nach der DE 10 2008 001 121 A1 ist eine aktive Überwachung. Sie beinhaltet eine Anregung durch eine Testeinspritzung, die sowohl den Kraftstoffverbrauch als auch die Emissionen erhöht. Das Verfahren nach der DE 10 2008 040 737 A1 arbeitet zwar passiv, setzt aber einen sogenannten Beobachter voraus, dessen Applikation aufwendig ist. Außerdem zielen beide Verfahren primär auf die Erkennung von größeren Totzeitänderungen ab.

In einer noch nicht veröffentlichen Anmeldung der Anmelderin mit dem internen Aktenzeichen R.339892 ist ein weiteres Verfahren sowie eine Vorrichtung zur Durchführung des Verfahrens zur Dynamiküberwachung von Gas-Sensoren einer Brennkraftmaschine beschrieben, welche beispielsweise als Abgassonden im Abgaskanal einer Brennkraftmaschine als Teil eines Abgasüberwachungs- und -minderungssystems oder als Gaskonzentrationssensoren in einer Zuluftführung der Brennkraftmaschine angeordnet sind, wobei die Gas-Sensoren abhängig von Geometrie, Messprinzip, Alterung oder Verschmutzung ein Tiefpassverhalten aufweisen, wobei bei einer Änderung der zu erfassenden Gaszustandsgröße auf Grund eines Vergleiches eines modellierten und eines gemessenen Signals eine Dynamikdiagnose durchgeführt wird und wobei das gemessene Signal ein Istwert eines Ausgangssignals des Gas-Sensors und das modellierte Signal ein Modellwert ist. Dabei ist vorgesehen, dass das Ausgangssignal des Gas-Sensors mit einem Hochpassfilter gefiltert und bei einer Änderung der zu messenden Gaszustandsgröße, wie auch einer Gaskonzentration, höherfrequente Signalanteile ausgewertet werden. Eine Änderung kann dabei durch eine Anregung der Brennkraftmaschine erfolgen. Mit diesem Verfahren lassen sich Änderungen hinsichtlich der Dynamik bei Gas-Sensoren nachweisen und quantifizieren. Gas-Sensoren im Sinne der Erfindung sind Sensoren, die Zustände eines Gases messen bzw. Änderungen detektieren können. Der Zustand des Gases kann dabei durch eine Temperatur des Gases, einen Gasdruck, einen Gas-Massenstrom und/ oder eine Konzentration eines bestimmten Gasanteils, z.B. einen Sauerstoffgehalt oder $NO_x$-Gehalt, beschrieben sein. Gas-Sensoren weisen ein typisches Tiefpassverhalten auf, das u.a. von der Geometrie ihres Aufbaus abhängt. Zudem können derartige Sensoren aufgrund von Alterung oder äußeren Einflüssen (z.B. infolge Verrußung bei Dieselmotoren) ihr Ansprechverhalten ändern.

Diese Methode der Dynamikdiagnose ist grundsätzlich geeignet, um eine T63-Filterzeitkonstante von Sensoren im Luft- und Abgassystem von Brennkraftmaschinen zu überwachen bzw. zu identifizieren. Hierzu vergleicht die Funktion die Signalenergien des Sensorsignals und eines modellbasierten Referenzsignals im Bereich höherer Frequenzen.

Prinzipiell könnte jedoch eine langsame, aber elektrisch schwingende Sonde mit dem Überwachungsprinzip gemäß der R.339892 fälschlicherweise als dynamisch in Ordnung erkannt werden. Dieser Fall ist z.B. vorstellbar, wenn eine Abgassonde stark verrußt ist, aber auf dem Kabelbaum eine elektromagnetische Störung einkoppelt oder die Auswerteschaltung einen elektrischen Fehler hat (Doppelfehler).

Es besteht daher die Aufgabe, eine Erweiterung einer Dynamiküberwachung für Gas-Sensoren bereitzustellen, die zusätzlich eine Schwingungserkennung bzw. eine Erkennung von elektrischen Störgrößen ermöglicht.

Es ist weiterhin Aufgabe der Erfindung, eine entsprechende Vorrichtung zur Durchführung des Verfahrens bereitzustellen.

Offenbarung der Erfindung

Die das Verfahren betreffende Aufgabe wird dadurch gelöst, dass in einem Stationärbetrieb der Brennkraftmaschine das Ausgangssignal des Gas-Sensors mit einem Hochpassfilter gefiltert und höherfrequente Signalanteile durch einen Vergleich mit dem entsprechend aufbereiteten Modellwert ausgewertet werden. So kann das Auftreten von vergleichsweise hohen Signalenergien nach der Hochpassfilterung während eines Stationärbetriebs auf eine Störung bzw. eine Einkopplung von Störgrößen hindeuten, wenn gleichzeitig in diesem Stationärbetrieb die ebenfalls hochpassgefilterten Signalenergien für den Modellwert vergleichsweise niedrige Werte annehmen. Mit dem Verfahren können somit elektrisch schwingende Gas-Sensoren bzw. Einkopplungen von Störgrößen oder Fehler in der Auswerteschaltung erkannt werden, wodurch Fehlinterpretationen bei einer Dynamikdiagnose minimiert werden können.

Damit zwischen dem Rausch-, Stör- und Nutzsignal unterschieden werden kann, ist eine Stationärerkennung erforderlich. Wird eine Änderungsgeschwindigkeit der zu messenden Gaszustandsgrößen ermittelt, kann daraus ein Stationärbetrieb der Brennkraftmaschine detektiert werden, da ein Stationärbetrieb nur geringe bis nahezu keine Änderungsgeschwindigkeiten für die zu messende Gaszustandsgröße aufweist. Dies kann mit einem Modell der zu messenden physikalischen Größe und einem weiteren Hochpassfilter geschehen. Der Zustand des Gases kann dabei durch eine Temperatur des Gases, einen Gasdruck, einen Gas-Massenstrom und/ oder eine Konzentration eines bestimmten Gasanteils, z.B. einen Sauerstoffgehalt oder $NO_x$-Gehalt, beschrieben sein. Im Falle eines $O_2$-Sensors genügt es beispielsweise, Luft- und Kraftstoffmasse in eine $O_2$-Konzentration umzurechnen und diese mit einem Tiefpassfilter zu verzögern, das einem funktionsfähigen Abgassensor entspricht. Dieses Tiefpassfilter ist dann mit dem gleichen Hochpassfilter in Reihe zu schalten wie die reale Abgassonde. Durch einen Vergleich der beiden Hochpassausgänge in Stationärphasen kann dann auf einen schwingenden Sensor, d.h. auf einen elektrischen Fehler geschlossen werden.

Eine bevorzugte Verfahrensvariante sieht vor, dass die Energie oder die Leistung der höherfrequenten Signalanteile des Gas-Sensors und von entsprechend hochpassgefilterten Ausgangssignalen aus einem Modell des Gas-Sensors mit Schwellwerten für die Energie oder der Leistung verglichen werden und anhand des Vergleichs auf das Vorhandensein von elektrischen Fehlern des Gas-Sensors oder auf das Auftreten von Schwingungen geschlossen wird.

Dabei kann vorgesehen sein, dass bei Überschreitung eines oberen Schwellwertes für die Energie oder der Leistung der höherfrequenten Signalanteile des Gas-Sensors und gleichzeitiger Unterschreitung eines unteren Schwellwertes für die Energie oder der Leistung der höherfrequenten Signalanteile des aus dem Modell bestimmten modellierten Signals auf einen elektrisch schwingenden Gas-Sensor geschlossen wird. Bei der Auswertung der hochpassgefilterten Signalenergien $\Phi_S$ für den Gas-Sensor und $\Phi_M$ für das modellierte Signal gilt dementsprechend: wenn die Energie $\Phi_M$ des Modellpfades kleiner ist als eine untere Schwelle $\Phi_{unten}$ und gleichzeitig die Energie $\Phi_S$ des Sensorpfades größer ist als eine obere Schwelle $\Phi_{oben}$, dann kann man dies so interpretieren, dass der Motorbetriebspunkt ungefähr konstant ist und das Sensorsignal dennoch stark schwankt. In diesem Fall kann auf einen schwingenden Sensor geschlossen werden.

Es ist dabei vorteilhaft, wenn eine im Modell hinterlegte Modellzeitkonstante $T_M$ der eines nominellen Gas-Sensors entspricht und/ oder diese sowie die Schwellwerte in Abhängigkeit von den Gaszustandsgrößen adaptiert werden. Die Kenngrößen für die Dynamik eines Gas-Sensors, beispielsweise einer Abgassonde, hängen i.d.R. von Abgasmassenstrom, Abgasvolumenstrom oder Abgasgeschwindigkeit ab. Daher ist es zweckmäßig, auch die Modellzeitkonstante $T_M$ sowie die Schwellwerte abhängig von einer solchen Zustandsgröße des Abgases nachzuführen.

In einer bevorzugten Verfahrensvariante wird die Integration der höherfrequenten Signalanteile des Sensorsignals und des modellierten Signals ereignisgesteuert oder bei erkanntem Stationärbetrieb der Brennkraftmaschine gestartet und zeit- oder ereignisgesteuert, d.h. nach einer festen Integrationsdauer T oder bei erkanntem Betriebspunktwechsel, beendet. Bei einer reinen Zeitsteuerung muss ggf. das Ergebnis bei einer Betriebspunktänderung innerhalb der Integrationsdauer T verworfen werden.

Im Falle der Ereignissteuerung hängen die Signalenergien $\Phi_S$ für den Gas-Sensor und $\Phi_M$ für das modellierte Signal stark von der aktuellen Integrationsdauer $T_{aktuell}$ ab. Es ist daher in einer Verfahrensvariante vorgesehen, im Falle einer Ereignissteuerung die Schwellwerte abhängig von einer aktuellen Integrationsdauer $T_{aktuell}$, welche von einer typischen Integrationsdauer $T_{norm}$ abweicht, anzupassen. Dies kann beispielsweise dadurch geschehen, dass die Schwellwerte $\Phi_{unten}$ und $\Phi_{oben}$ für eine typische Integrationsdauer $T_{norm}$ appliziert und gemäß

$$\Phi_{unten,\ aktuell} \quad = \quad \Phi_{unten,\ norm} (T_{aktuell} / T_{norm}) \qquad (1a)$$

$$\Phi_{oben,\ aktuell} \quad = \quad \Phi_{oben,\ norm} (T_{aktuell} / T_{norm}) \qquad (1b)$$

an die aktuelle Integrationsdauer $T_{aktuell}$ angepasst werden.

Eine alternative Verfahrensvariante besteht darin, nicht Signalenergien $\Phi_M$ bzw. $\Phi_S$ zu vergleichen, sondern dass eine mittlere Signalleistung der höherfrequenten Signalanteile des Sensorsignals und des modellierten Signals ausgewertet werden und der Vergleich mit applizierbarem Leistungsschwellwert erfolgt. Die mittlere Signalleistungen ergeben sich demnach gemäß $P_M = \Phi_M/T$ und $P_S = \Phi_S/T$. In diesem Fall ist das Integrationsergebnis durch die jeweilige Integrationsdauer $T$ zu dividieren. Die Schwellwerte sind dann durch entsprechende Leistungsschwellwerte $P_{unten}$ und $P_{oben}$ zu ersetzen. Diese Vorgehensweise hat den Vorteil, dass die Schwellwerte nicht an die tatsächliche Integrationsdauer angepasst werden müssen.

Um die Trennschärfe der Diagnose und das Signal-Rausch-Verhältnis zu verbessern, ist vorgesehen, dass bei der Hochpassfilterung zusätzlich das Signal des Gassensors und/ oder des modellierten Signals mit Filtereinheiten bzw. Filterfunktionen gefiltert werden, die in ihrem Kennlinienverlauf Unempfindlichkeits- oder Totzonen im Bereich kleiner Eingangsgrößen aufweisen, wobei diese zusätzlich in ihrem Kennlinienverlauf Sprünge aufweisen können. Hiermit werden insbesondere kleine Signalgrößen, die aufgrund von Rauschen auftreten können, unterdrückt. Der Einsatz dieser zusätzlichen Filtereinheiten bzw. Filterfunktionen bei der Hochpassfilterung ist nicht nur im Sensorpfad sinnvoll, sondern auch im Modellpfad, da das Modell typischerweise andere Sensorsignale verwendet, die ebenfalls verrauscht sein können. Wenn die potentiellen elektrischen Fehler bekannt sind und niedrigere Frequenzen aufweisen als das Rauschen, kann auch ein Software-Tiefpassfilter vor den Nichtlinearitäten im Signalpfad eingesetzt werden.

In entsprechenden Varianten für diese Filtereinheiten bzw. Filterfunktionen kann vorgesehen sein, dass die Kennlinienverläufe dieser Filtereinheiten mit der Funktionalität des Quadrierens der Signale zur Berechnung der Signalleistung der höherfrequenten Signalanteile zusammengefasst oder der Betrag des Hochpasssignalausgangs verwendet oder auch die Betragsbildung mit der Unempfindlichkeits- oder Totzone in einer Kennlinie der Filtereinheiten zusammengefasst werden.

Das erfinderische Überwachungsverfahren kann besonders vorteilhaft bei GasSensoren eingesetzt werden, die als Gas-Drucksensoren, Gas-Temperatursensoren, Gas-Massenstromsensoren oder Gaskonzentrationssensoren als Abgassonden im Abgaskanal der Brennkraftmaschine als Teil eines Abgasüberwachungs- und -minderungssystems oder in einer Zuluftführung der Brennkraftmaschine, z.B. im Ansaugkrümmer, verwendet werden, um Gaszustandsgrößen bzw. Konzentrationen zu erfassen. Diese emissionsrelevanten Gas-Sensoren müssen aufgrund der eingangs erwähnten Anforderungen hinsichtlich ihrer Dynamik und generellen Funktion überwacht werden. Eine Erkennung von unzulässigen Schwingungen ist hierbei hinsichtlich eines korrekten Diagnoseergebnisses von großer Bedeutung. So kann z.B. das Ansprechverhalten eines Gas-Drucksensors überwacht werden und ein Nachlassen der Dynamik detektiert werden, wenn beispielsweise die Anbindung des Gas-Drucksensors an einen Ansaugkrümmer verstopft oder abgeknickt ist. Gas-Temperatursensoren oder Gas-Massenstromsensoren können beispielsweise als Heißfilm-Luftmassenmesser innerhalb einer Zuluftführung der Brennkraftmaschine ausgeführt sein, bei denen infolge einer Verschmutzung ein Dynamikverlust zu verzeichnen sein kann. Störsignaleinkopplungen, die z.B. zu Schwingungen führen können, können mit dem Verfahren erkannt werden. Sofern für die Signale solcher Sensoren ein geeignetes Modell angegeben werden kann, kann das erfindungsgemäße Verfahren, wie zuvor in seinen Verfahrensvarianten beschrieben, vorteilhaft angewendet werden.

Als Gas-Sensoren kommen insbesondere Abgassonden in Form von Breitband-Lambdasonden (LSU-Sonden) oder $NO_x$-Sensoren in Betracht, mit denen ein Sauerstoffgehalt in einem Gasgemisch bestimmt werden kann. Für eine als Breitband-Lambdasonde oder stetige Lambdasonde ausgeführte Abgassonde wird zur Diagnose vorzugsweise die gemessene Sauerstoffkonzentration mit einer modellierten Sauerstoffkonzentration entsprechend den zuvor beschriebenen Verfahrensvarianten verglichen. Alternativ kann für diesen Vergleich der reziproke Lambdawert verwendet werden, da er näherungsweise proportional zur Sauerstoffkonzentration ist. Ebenfalls geeignet sind elektrische Größen, die proportional zur Sauerstoffkonzentration sind, d.h. eine Spannung oder ein Strom im Sensor bzw. im zugehörigen Schaltkreis. Das zum Vergleich herangezogene Modellsignal muss dann entsprechend umgerechnet werden. Für einen Stickoxid-Sensor wird als Istwert das Ausgangssignal des Stickoxid-Sensors ausgewertet, wobei der Modellwert aus einem modellierten $NO_x$-Wert bestimmt wird. Diese Überwachung lässt sich daher besonders vorteilhaft bei OttoMotoren oder bei Mager-Motoren anwenden, deren Abgasreinigungsanlage einen Katalysator und/ oder Einrichtungen zur Stickoxid-Reduktion aufweisen. Auftretende Schwingungen können hierbei besonders negative Folgen hinsichtlich des Betriebs der Abgasreinigungsanlagen haben. Bei Gas-Sensoren, die nach einer Abgasreinigungsanlage verbaut sind, muss der Einfluss der Abgasreinigung auf die interessierende Gaskonzentration im Modell berücksichtigt werden. Alternativ ist vorstellbar, die Diagnose nur in Phasen durchzuführen, in denen die Abgasreinigung keinen Einfluss auf die interessierende Gaskonzentration hat.

Eine weitere Anwendung des Verfahrens mit seinen zuvor beschriebenen Varianten kann generell bei Prozessen mit mindestens einem Sensor vorgesehen sein, bei denen sich der Prozess durch einen Filter erster Ordnung mit Zeitkonstante sowie ggf. mit einer Totzeit approximieren lässt und das Verhalten eines verlangsamten Sensors durch eine vergrößerte Filterzeitkonstante beschrieben werden kann. Grundsätzlich ergeben sich auch hierbei die zuvor beschrieben Vorteile hinsichtlich einer Beurteilung des Ansprechverhaltens des Sensors. Zudem kann ein solcher Prozess hinsichtlich seines Regelverhaltens verbessert werden, indem sein Regler an die veränderte Zeitkonstante angepasst wird.

Wird, wie dies eine bevorzugte Verfahrensvariante vorsieht, die Funktionalität des zuvor beschriebenen Überwachungsverfahrens mit Verfahren zur Dynamikdiagnose von Gas-Sensoren kombiniert, bei denen zur Dynamikdiagnose eine Auswertung höherfrequenter Signalanteile bei einer Änderung der zu messenden Gaszustandsgröße durchgeführt wird, kann die Robustheit des Dynamikdiagnoseverfahrens deutlich erhöht werden, da Fehlinterpretationen infolge von Störsignaleinkopplungen, wie sie eingangs erwähnt wurden, weitgehend vermieden werden können. Der zusätzliche Applikationsaufwand ist in diesem Fall vergleichsweise gering, da seitens der Dynamikdiagnose bereits alle wesentlichen Funktionselemente vorgesehen sind, auf die die Überwachungsfunktion zugreifen kann. Hierbei kann die Dynamikdiagnose in Betriebsphasen mit Änderungen der zu messenden Gaszustandsgröße und eine Überwachung auf Schwingungen des Systems oder auf mögliche elektrische Fehler in Stationärphasen stattfinden. Als ein mögliches Dynamikdiagnoseverfahren bietet sich das eingangs erwähnte, noch nicht veröffentlichte Verfahren gemäß der Schrift R.339892 an.

Die die Vorrichtung betreffende Aufgabe wird dadurch gelöst, dass zur Durchführung des erfindungsgemäßen Verfahrens eine Überwachungseinheit vorgesehen ist, die Hochpassfilter zur Auswertung höherfrequenter Signalanteile sowie mindestens ein Modell für den Gas-Sensor und Berechnungseinheiten und zusätzliche Filtereinheiten bzw. Filterfunktionen zur Durchführung der Überwachung gemäß den zuvor beschriebenen Verfahren mit seinen Varianten aufweist. Die Überwachungseinheit kann dabei integraler Bestandteil der übergeordneten Motorsteuerung sein. Die Funktionalität des Verfahrens kann dabei zumindest teilweise softwarebasiert in dieser implementiert sein.

Die Erfindung wird im Folgenden anhand eines in den Figuren dargestellten Ausführungsbeispiels näher erläutert. Es zeigen:

Figur 1 in schematischer Darstellung das technische Umfeld, in dem das erfindungsgemäße Verfahren angewendet werden kann,

Figur 2 ein Blockdiagramm einer Dynamikdiagnoseschaltung mit einem Vergleich einer Signalenergie, welche als Basis einer Plausibilitätsprüfung zur Detektion von Schwingungen bzw. von eingekoppelten elektrischen Störgrößen herangezogen werden kann,

Figur 3a und 3b einen Hochpassfilter als Bestandteil der Dynamikdiagnoseschaltung mit einer Filtereinheit, welche einen Unempfindlichkeitsbereich ohne (Figur 3a) und mit Sprung (Figur 3b) aufweist,

Figur 4a und 4b zwei alternative Ausführungen des Unempfindlichkeitsbereichs ohne Sprung,

Figur 5a und 5b zwei alternative Ausführungen des Unempfindlichkeitsbereichs mit Sprung und

Figur 6 ein Blockdiagramm einer zur in Figur 2 alternativen Dynamikdiagnoseschaltung mit einem Vergleich einer mittleren Signalleistung.

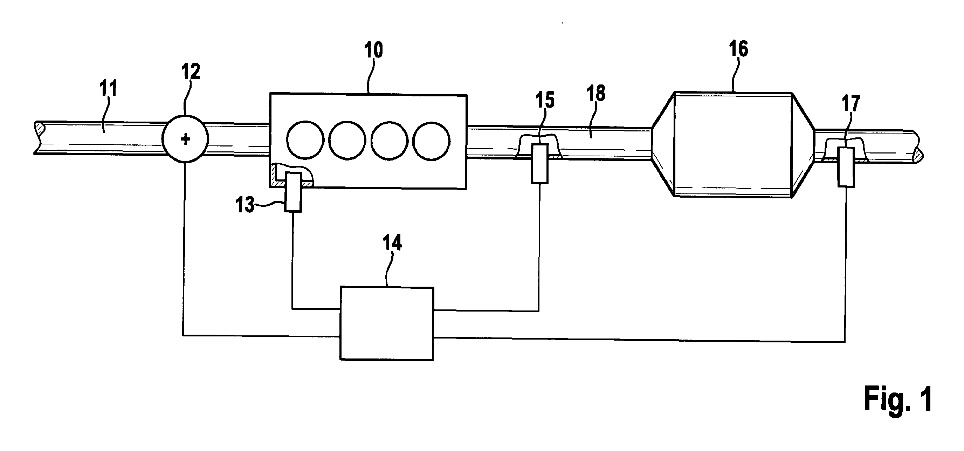

Figur 1 zeigt schematisch an einem Beispiel eines Otto-Motors das technische Umfeld, in dem das erfindungsgemäße Verfahren zur Diagnose einer Abgassonde 15 eingesetzt werden kann. Einer Brennkraftmaschine 10 wird Luft über eine Luftzuführung 11 zugeführt und deren Masse mit einem Luftmassenmesser 12 bestimmt. Der Luftmassenmesser 12 kann als Heißfilm-Luftmassenmesser ausgeführt sein. Das Abgas der Brennkraftmaschine 10 wird über einen Abgaskanal 18 abgeführt, wobei in Strömungsrichtung des Abgases hinter der Brennkraftmaschine 10 eine Abgasreinigungsanlage 16 vorgesehen ist. Die Abgasreinigungsanlage 16 umfasst üblicherweise mindestens einen Katalysator.

Zur Steuerung der Brennkraftmaschine 10 ist eine Motorsteuerung 14 vorgesehen, die zum einen der Brennkraftmaschine 10 über eine Kraftstoffdosierung 13 Kraftstoff zuführt und der zum anderen die Signale des Luftmassenmessers 12 und der in dem Abgaskanal 18 angeordneten Abgassonde 15 sowie einer in der Abgasableitung 18 angeordneten Abgassonde 17 zugeführt werden. Die Abgassonde 15 bestimmt im gezeigten Beispiel einen Lambda-Istwert eines der Brennkraftmaschine 10 zugeführten Kraftstoff-Luft-Gemischs. Sie kann als Breitband-Lambdasonde oder stetige LambdaSonde ausgeführt sein. Die Abgassonde 17 bestimmt die Abgaszusammensetzung nach der Abgasreinigungsanlage 16. Die Abgassonde 17 kann als Sprungsonde oder Binärsonde ausgebildet sein.

Hinsichtlich einer verbesserten Dynamiküberwachung der Abgassonde 15 kann vorgesehen sein, dass Hoch- und Tiefpassfilter genutzt werden, um bei einem Lastwechsel der Brennkraftmaschine 10 zu prüfen, ob die höherfrequenten Anteile einer Konzentrationsänderung von der Abgassonde 15 noch erkannt werden. Derartige Gas-Sensoren weisen ein typisches Tiefpassverhalten auf, das u.a. von der Geometrie ihres Schutzrohres abhängt. Bei Dieselmotoren kann zudem ein solches Schutzrohr verrußen, wodurch die Bandbreite des Sensors abnimmt. Im Zeitbereich äußert sich die abnehmende Grenzfrequenz in einer größeren Anstiegszeit, d.h. bei unveränderter Anregung werden die Signalflanken flacher. Schaltet man daher mit der Sonde einen geeigneten Hochpassfilter in Reihe, dann kann man bei steilen Lastwechseln am Ausgangssignal des Hochpasses erkennen, ob die Grenzfrequenz des Tiefpasses größer oder kleiner als die Grenzfrequenz des Hochpasses ist. Durch Auswertung dieser höherfrequenten Signalenergien kann auf die Dynamik des Sensors geschlossen werden, wie dies in der Schrift R.339892 beschrieben ist.

Wenn die Signalenergie einer Abgassonde 15 nach Hochpassfilterung im Stationärbetrieb einen unplausibel hohen Wert annimmt, kann zudem auf eine elektrische Oszillation oder Einkopplung von Störsignalen geschlossen werden. Als Referenz für die Plausibilitätsprüfung kann, wie in der R.339892 beschrieben, ein Modell herangezogen werden.

Die vorliegende Erfindung nutzt eine aus der R.339892 bekannte Filteranordnung, um in stationären Motorbetriebsphasen nach hochfrequenten Anteilen im Sensorsignal zu suchen, die eigentlich nicht vorhanden sein dürften. Schaltet man mit der Abgassonde 15 ein geeignetes Hochpassfilter in Reihe, dann werden der Gleichanteil und niederfrequente Anteile des Messsignals unterdrückt. Bei stationärem Motorbetrieb darf demnach nur das Messrauschen zur Ausgangsleistung des Hochpassfilters beitragen.

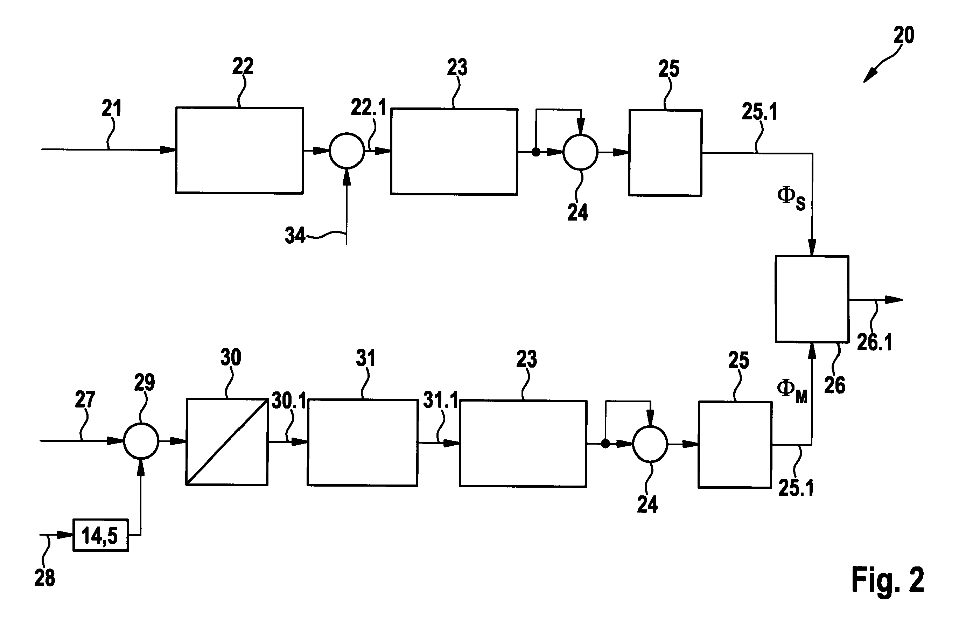

Figur 2 zeigt in einem Blockdiagramm 20 die Grundstruktur der Dynamiküberwachung aus der R.339892. Dargestellt ist im oberen Teil ein Pfad für eine mit der Abgassonde 15 gemessene Sauerstoffkonzentration 21. Infolge einer realen Gaslaufzeit und Sondenverzögerung 22, welche durch eine Totzeit $T_t$ bzw. einen Tiefpassfilter 1. Ordnung mit einer Sonden-Zeitkonstante $T_S$ beschrieben werden können, ergibt sich aus der realen Sauerstoffkonzentration 21 ein Sauerstoff-Sondensignal 22.1. Die Übertragungsfunktion der Sonden und- Gaslaufzeit 22 ergibt sich durch folgende Beziehung, wobei $K_S$ einen Verstärkungsfaktor für die Sonde darstellt:

$$G(j\omega) = K_S \exp(- T_t\, j\omega) / (T_S\, j\omega + 1) \qquad\qquad (2)$$

$K_S$ entspricht in der Regel dem multiplikativen oder Steigungsfehler der Sonde, der von Produktionsstreuung und Alterung herrührt. Wird jedoch als Sondensignal nicht die Sauerstoffkonzentration verwendet, sondern eine dazu proportionale Größe, dann ist $K_S$ ein entsprechender Übertragungsbeiwert zur Umrechnung des Sondensignals in eine Sauerstoffkonzentration und kann auch dimensionsbehaftet sein. Anschließend wird das Sauerstoff-Sondensignal 22.1 mit einem Hochpass 23 gefiltert und mit einem Multiplizierer 24 quadriert, was ein Signal liefert, welches einer Signalleistung entspricht. Dieses Signal wird anschließend mittels eines Integrators 25 integriert, so dass man eine Signalenergie 25.1 der höherfrequenten Energieanteile des gemessenen Sauerstoffgehaltes erhält. In einer nachgeschalteten Auswerteeinheit 26 ergibt sich aus einem Vergleich mit einem entsprechend aufbereiteten Signal für einen modellhaft bestimmten Wert ein Statuswert 26.1, welcher zur Diagnose verwendet werden kann.

In Folge von z.B. Einkopplungen auf dem Kabelbaum oder durch elektrische Fehler der Auswerteschaltung können, wie dies Figur 2 zeigt, elektrische Störgrößen 34 im Sensorpfad eingekoppelt werden. Das Tiefpassverhalten des Sensors hat auf das Spektrum der Störgrößen naturgemäß keinen Einfluss. Dementsprechend hat beispielsweise eine Verrußung des Sensors auch keinen Einfluss auf die Störempfindlichkeit des Sensorpfades.

Der Hochpass 23 kann im einfachsten Fall als Hochpass 1. Ordnung ausgelegt sein, dessen Übertragungsfunktion durch die Beziehung

$$G(j\omega) = T_F\, j\omega / (T_F\, j\omega + 1) \qquad\qquad (3)$$

beschrieben werden kann mit $T_F$ als Grenzfrequenz des Filters. Übersteigt die Grenzfrequenz der Abgassonde 15 die Grenzfrequenz $T_F$ des Hochpasses 23, dann verhält sich die Serienschaltung wie ein Bandpass, d.h. die hohen Frequenzen des Eingangsspektrums der Abgassonde 15 werden noch durchgelassen und können im Ausgangsspektrum detektiert werden. Fällt dagegen die Grenzfrequenz der Abgassonde 15 infolge eines Dynamikverlustes unter die Grenzfrequenz $T_F$ des Hochpasses 23, dann sperrt die Reihenschaltung sämtliche Frequenzen, so dass im Ausgangsspektrum keinerlei Frequenzanteile mehr gemessen werden können.

Prinzipiell beschränkt sich die Erfindung dabei nicht auf Hochpassfilter 1. Ordnung. Vielmehr können auch beliebig andere Hochpassfilter zum Einsatz kommen. Ebenso ist die Überwachungsmethode anwendbar, wenn die Tiefpassfilter einschließlich der Abgassonde 15 selbst anders parametriert werden, z.B. mit einer Grenzfrequenz anstelle der Zeitkonstanten, oder eine höhere Ordnung besitzen.

Damit mit dem Verfahren aus der R.339892 zwischen einer langsamen Abgassonde 15 und einer unzureichenden Anregung unterschieden werden kann, muss die Änderungsgeschwindigkeit der Abgaszusammensetzung beurteilt werden, was beispielsweise im Falle einer Breitband-Lambdasonde anhand von Luft- und Kraftstoffmassenänderung geschehen kann. Dies kann mit einer ähnlichen Reihenschaltung von Filtern geschehen. Im Falle einer Breitband-Lambdasonde müssen hierfür nur die vorstehenden Massen in eine $O_2$-Konzentration umgerechnet und mit einem Tiefpassfilter verzögert werden, der einem funktionsfähigen Abgassensor entspricht. Dieser Tiefpassfilter ist dann mit einem Hochpass in Reihe zu schalten, der die gleiche Übertragungsfunktion besitzt wie der der realen Sonde. Durch einen Vergleich der beiden HochpassAusgänge kann dann auf die Funktionstüchtigkeit des realen Sensors geschlossen werden. Im Falle einer anderen Gaskomponente kann es erforderlich werden, ein zusätzliches Rohemissionsmodell zu verwenden.

Die Aufbereitung des modellhaft bestimmten Energiewertes wird im unteren Teil des Blockdiagramms 20 in Figur 2 gezeigt. Aus einer Luftmasse 27 $m_L$ und einer Soll-Kraftstoffmasse 28 $m_K$ für die Kraftstoffdosierung 13 wird nach stöchiometrischer Korrektur in einer Divisionseinheit 29 ein Quotient gebildet und ein Lambdawert errechnet. Die Kraftstoffmasse 28 kann sich aus dem Drehmomentwunsch ergeben, den der Fahrer vorgibt und der in eine Kraftstoffmenge umgerechnet wird. In einer Umrechnungseinheit 30 wird aus dem Lambdawert ein kalkulierter Sauerstoffgehalt 30.1 bestimmt. Gemäß einem Modell 31 wird mit der Übertragungsfunktion

$$G(j\omega) = \exp(- T_{tM}\, j\omega) / (T_M\, j\omega + 1) \qquad\qquad (4)$$

ein modellierter Sauerstoffgehalt 31.1 berechnet, wobei $T_{tM}$ eine Modell-Totzeit und $T_M$ eine Modell-Zeitkonstante darstellt.

Anschließend wird der modellierte Sauerstoffgehalt 31.1 mit einem weiteren Hochpass 23, dessen Übertragungsfunktion im einfachsten Fall der des Hochpasses 1. Ordnung entspricht, gefiltert und mit einem weiteren Multiplizierer 24 quadriert, was ein Signal liefert, welches einer Signalleistung entspricht. Dieses Signal wird anschließend mittels eines weiteren Integrators 25 integriert, so dass man eine Signalenergie 25.1 für die höherfrequenten Energieanteile des modellierten Sauerstoffgehalts erhält.

Da der Hochpass 23 den Gleichanteil und die niederfrequenten Anteile unterdrückt, liefern nur die höherfrequenten Anteile des jeweiligen $O_2$-Signals 22.1, 31.1 einen Beitrag. Im Stationärbetrieb sollten also die beiden Hochpass-Ausgangssignale $Y_S$ für das Sensorsignal und $Y_M$ für das Modellsignal verschwinden, wenn man vom Rauschen absieht. Demnach sollten auch die beiden Signalenergien 25.1

$$\Phi_M = \int_0^T Y_M^2(t)\,dt \qquad\qquad (5a)$$

$$\Phi_S = \int_0^T Y_S^2(t)\,dt \qquad\qquad (5b)$$

im Stationärbetrieb sehr niedrige Werte annehmen, wobei T die Integrationsdauer darstellt.

Aus dem Vergleich der beiden Signalenergien 25.1 in der Auswerteeinheit 26 kann nun auf einen elektrischen Fehler der Abgassonde 15 geschlossen werden. Wenn die Energie $\Phi_M$ des Modellpfades kleiner ist als eine untere Schwelle $\Phi_{unten}$ und gleichzeitig die Energie $\Phi_S$ des Sensorpfades größer ist als eine obere Schwelle $\Phi_{oben}$, dann kann man dies so interpretieren, dass der Motorbetriebspunkt ungefähr konstant ist und das Sensorsignal dennoch stark schwankt, was auf eine elektrisch schwingende Abgassonde 15 hindeutet. Zusammengefasst gilt:

$$\Phi_M < \Phi_{unten} \quad \text{und} \quad \Phi_S > \Phi_{oben} \quad \rightarrow \quad \text{Sensor schwingt}$$

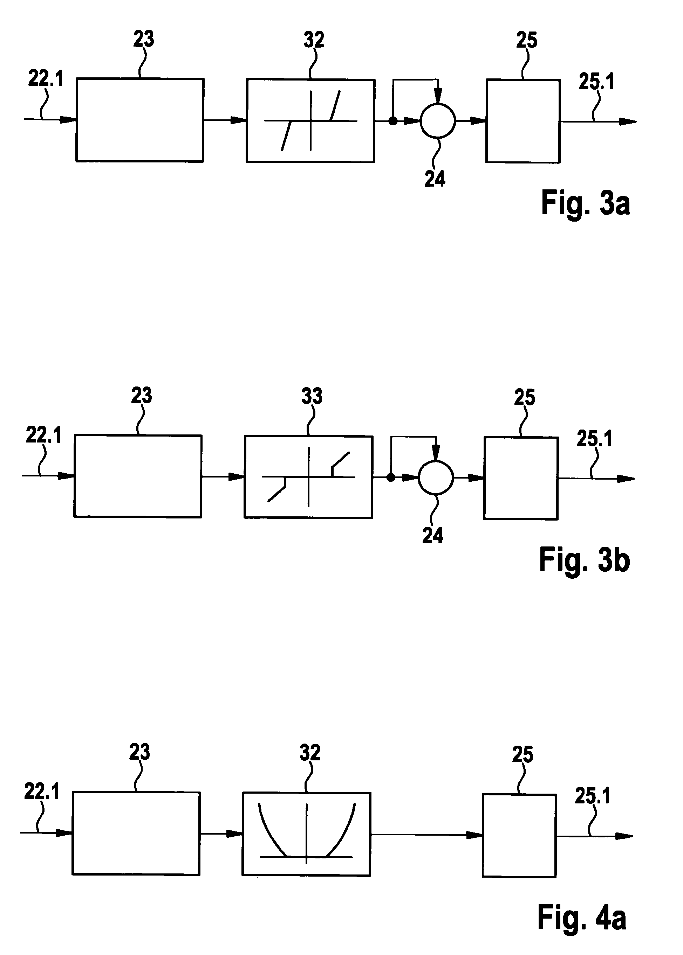

Um die Trennschärfe der Diagnose zu verbessern, ist es empfehlenswert, eine sogenannte Unempfindlichkeits- oder Totzone einzusetzen, wie sie zur Rauschfilterung üblich ist. Diese unterdrückt in einem applizierbaren Bereich kleine Werte ihrer Eingangsgröße. Diese Unempfindlichkeitszone kann beispielsweise durch die in den Figuren 3a, 3b, 4a, 4b sowie 5a und 5b dargestellten zusätzlichen Filtereinheiten 32, 33 bzw. Filterfunktionen mit ihren Kennlinien realisiert werden, wobei die Kennlinien auch einen Sprung aufweisen können (jeweils Filtereinheit 33).

Figur 3a zeigt ausschnittsweise den Sensorpfad. Das Sauerstoff-Sondensignal 22.1 wird mit dem Hochpass 23 gefiltert, wobei dieser eine Filtereinheit mit einem Unempfindlichkeitsbereich (Totzone) ohne Sprung 32 aufweist. Das so gefilterte Signal wird anschließend mittels des Multiplizierers 24 quadriert und mit dem Integrator 25 integriert, so dass man die Signalenergie 25.1 ausgangsseitig erhält. Wie aus der Kennlinie ersichtlich, werden hierbei kleine Signalgrößen um den Nullpunkt unterdrückt.

Figur 3b zeigt eine zur Figur 3a alternative Anordnung, bei der eine Filtereinheit 33 mit einem Unempfindlichkeitsbereich mit Sprung eingesetzt ist, was zudem die Trennschärfe erhöht.

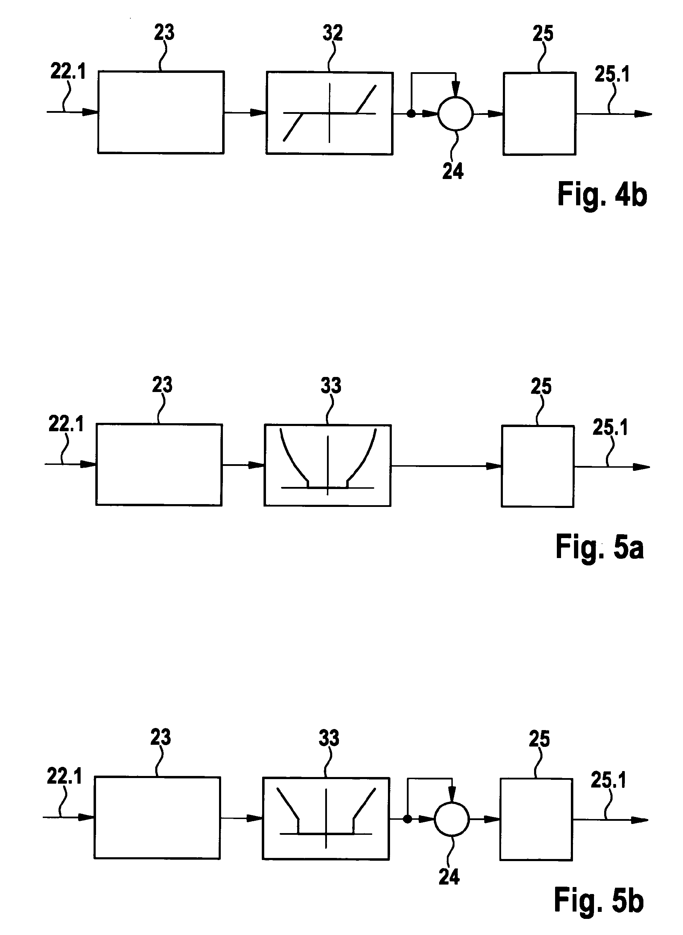

Die Unempfindlichkeits- oder Totzone kann mit dem Quadrieren in einer Kennlinie zusammengefasst sein. Ebenso kann auch der Betrag des Hochpassausgangssignals verwendet und auch die Betragsbildung mit der Totzone in einer Kennlinie zusammengefasst sein. Diese Varianten werden in den Figuren 4a, 4b sowie 5a und 5b gezeigt.

Figur 4a zeigt eine Filtereinheit 32 mit einem Unempfindlichkeitsbereich (Totzone) ohne Sprung, bei der die Kennlinie als Parabel ausgelegt ist, so dass der Multiplizierer 24 entfallen kann, da die parabelförmige Übertragungsfunktion der Filtereinheit 32 bereits ausgangsseitig ein quadriertes Signal erzeugt.

Figur 4b zeigt eine Filtereinheit 32 mit einem Unempfindlichkeitsbereich (Totzone) ohne Sprung, bei der die Betragsbildung mit der Totzone in einer Kennlinie zusammengefasst ist.

Figur 5a und 5b zeigen jeweils zu Figur 4a und 4b alternative Anordnungen, bei der die Kennlinie der Filtereinheit 33 beim Unempfindlichkeitsbereich einen Sprung aufweist.

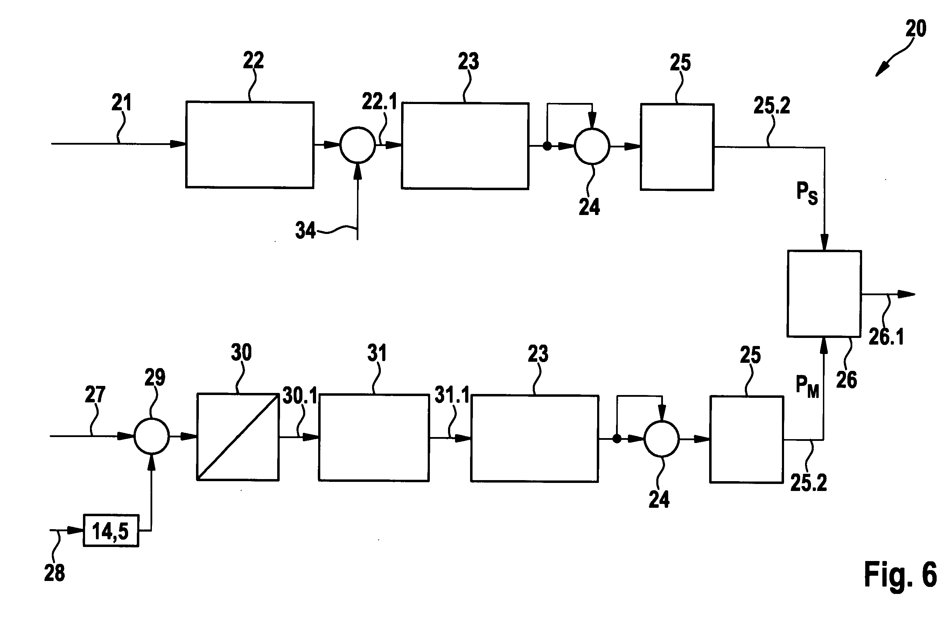

Figur 6 zeigt eine entsprechende Anordnung, bei der, abweichend zur in Figur 2 gezeigten Anordnung, der Integrator 25 jeweils das Ergebnis durch die Integrationszeit T dividiert und somit zum Vergleich die mittleren Signalleistungen 25.2 für $P_S$ und $P_M$ zur Verfügung stehen. Der Vergleich erfolgt dann gemäß

$$P_M < P_{unten} \quad und \quad P_S > P_{oben} \quad \rightarrow \quad Sensor\ schwingt$$

Ein derartiger Leistungsvergleich ist selbstverständlich mit allen zuvor genannten Unempfindlichkeitsbereichen gemäß den Figuren 3a, 3b, 4a, 4b sowie 5a und 5b kombinierbar.

Der Einsatz ist sowohl bei Benzin- oder Diesel-Brennkraftmaschinen denkbar, bei denen eine Schwingungserkennung von Gassensoren erforderlich ist, wie dies insbesondere bei abgasrelevanten Sensoren der Fall sein kann. Diese Überwachungsfunktion kann eigenständig eingesetzt oder mit Dynamikdiagnosefunktionen, wie sie beispielsweise in der R.339892 beschrieben sind, kombiniert werden.

**Patentansprüche**

1. Verfahren zur Überwachung von Gas-Sensoren einer Brennkraftmaschine (10), wobei die Gas-Sensoren abhängig von Geometrie, Messprinzip, Alterung oder Verschmutzung ein Tiefpassverhalten aufweisen, wobei bei der zu messenden Gaszustandsgröße ein Vergleich eines modellierten und eines gemessenen Signals eine Diagnose durchgeführt wird und wobei das gemessene Signal ein Istwert eines Ausgangssignals des Gas-Sensors und das modellierte Signal ein Modellwert ist, **dadurch gekennzeichnet, dass** in einem Stationärbetrieb der Brennkraftmaschine (10) das Ausgangssignal des Gas-Sensors mit einem Hochpassfilter (23) gefiltert und höherfrequente Signalanteile durch einen Vergleich mit dem entsprechend aufbereiteten Modellwert ausgewertet werden.

2. Verfahren nach Anspruch 1, **dadurch gekennzeichnet, dass** eine Änderungsgeschwindigkeit der zu messenden Gaszustandsgrößen ermittelt und daraus ein Stationärbetrieb der Brennkraftmaschine (10) detektiert wird.

3. Verfahren nach Anspruch 1 oder 2, **dadurch gekennzeichnet, dass** die Energie oder die Leistung der höherfrequenten Signalanteile des Gas-Sensors und von entsprechend hochpassgefilterten Ausgangssignalen aus einem Modell (31) des GasSensors mit Schwellwerten für die Energie oder der Leistung verglichen werden und anhand des Vergleichs auf das Vorhandensein von elektrischen Fehlern des GasSensors oder auf das Auftreten von Schwingungen geschlossen wird.

4. Verfahren nach Anspruch 3, **dadurch gekennzeichnet, dass** bei Überschreitung eines oberen Schwellwertes für die Energie oder der Leistung der höherfrequenten Signalanteile des Gas-Sensors und gleichzeitiger Unterschreitung eines unteren Schwellwertes für die Energie oder der Leistung der höherfrequenten Signalanteile des aus dem Modell (31) bestimmten modellierten Signals auf einen elektrisch schwingenden Gas-Sensor geschlossen wird.

5. Verfahren nach einem der Ansprüche 1 bis 4, **dadurch gekennzeichnet, dass** eine im Modell (31) hinterlegte Modellzeitkonstante $T_M$ der eines nominellen Gas-Sensors entspricht und/ oder diese sowie die Schwellwerte in Abhängigkeit von den Gaszustandsgrößen adaptiert werden.

6. Verfahren nach einem der Ansprüche 1 bis 5, **dadurch gekennzeichnet, dass** die Integration der höherfrequenten Signalanteile des Sensorsignals und des modellierten Signals ereignisgesteuert oder bei erkanntem Stationärbetrieb der Brennkraftmaschine (10) gestartet und zeit- oder ereignisgesteuert beendet wird.

7. Verfahren nach Anspruch 6, **dadurch gekennzeichnet, dass** im Falle einer Ereignissteuerung die Schwellwerte abhängig von einer aktuellen Integrationsdauer $T_{aktuell}$, welche von einer typischen Integrationsdauer $T_{norm}$ abweicht, angepasst werden.

8. Verfahren nach einem der Ansprüche 1 bis 6, **dadurch gekennzeichnet, dass** eine mittlere Signalleistung der höherfrequenten Signalanteile des Sensorsignals und des modellierten Signals ausgewertet werden und der Vergleich mit applizierbarem Leistungsschwellwert erfolgt.

9. Verfahren nach einem der Ansprüche 1 bis 8, **dadurch gekennzeichnet, dass** bei der Hochpassfilterung zusätzlich das Signal des Gassensors und/ oder des modellierten Signals mit Filtereinheiten (32, 33) oder Filterfunktionen gefiltert werden, die in ihrem Kennlinienverlauf Unempfindlichkeits- oder Totzonen im Bereich kleiner Eingangsgrößen aufweisen, wobei diese zusätzlich in ihrem Kennlinienverlauf Sprünge aufweisen können.

10. Verfahren nach Anspruch 9, **dadurch gekennzeichnet, dass** die Kennlinienverläufe dieser Filtereinheiten (32, 33) oder Filterfunktionen mit der Funktionalität des Quadrierens der Signale zusammengefasst oder der Betrag des Hochpasssignalausgangs verwendet oder auch die Betragsbildung mit der Unempfindlichkeits- oder Totzone in einer Kennlinie der Filtereinheiten (32, 33) zusammengefasst werden.

11. Verfahren nach einem der Ansprüche 1 bis 10, **dadurch gekennzeichnet, dass** als Gas-Sensoren Gas-Drucksensoren, Gas-Temperatursensoren, Gas-Massenstromsensoren oder Gaskonzentrationssensoren als Abgassonden (15) im Abgaskanal (18) der Brennkraftmaschine (10) als Teil eines Abgasüberwachungs- und - minderungssystems oder in einer Zuluftführung (11) der Brennkraftmaschine (10) verwendet werden.

12. Verfahren nach einem der Ansprüche 1 bis 11, **dadurch gekennzeichnet, dass** als Gas-Sensoren Abgassonden (15) in Form von Breitband-Lambdasonden oder $NO_x$-Sensoren verwendet werden, mit denen ein Sauerstoffgehalt in einem Gasgemisch bestimmt werden kann.

13. Verfahren nach einem der Ansprüche 1 bis 12, **dadurch gekennzeichnet, dass** das Überwachungsverfahren nach den Ansprüchen 1 bis 12 mit Verfahren zur Dynamikdiagnose von Gas-Sensoren kombiniert wird, bei denen zur Dynamikdiagnose eine Auswertung höherfrequenter Signalanteile bei einer Änderung der zu messenden Gaszustandsgröße durchgeführt wird.

14. Vorrichtung zur Überwachung von Gas-Sensoren im Abgaskanal einer Brennkraftmaschine (10) als Teil eines Abgasüberwachungs- und -minderungssystems oder in einer Zuluftführung der Brennkraftmaschine (10), wobei die Gas-Sensoren abhängig von Geometrie, Messprinzip, Alterung oder Verschmutzung ein Tiefpassverhalten aufweisen, wobei bei der zu messenden Gaszustandsgröße auf Grund eines Vergleiches eines modellierten und eines gemessenen Signals eine Diagnose in einer Überwachungseinheit durchgeführt wird und wobei das gemessene Signal ein Istwert eines Ausgangssignals des Gas-Sensors und das modellierte Signal ein Modellwert ist, **dadurch gekennzeichnet, dass** die Überwachungseinheit Hochpassfilter (23) zur Auswertung höherfrequenter Signalanteile sowie mindestens ein Modell (31) für den Gas-Sensor und Berechnungseinheiten und zusätzliche Filtereinheiten (32, 33) oder Filterfunktionen zur Durchführung der Überwachung nach den Ansprüchen 1 bis 13 aufweist.

**Claims**

1. Method for monitoring gas sensors of an internal combustion engine (10), wherein the gas sensors have a low-pass behaviour depending on the geometry, measuring principle, ageing or soiling, wherein diagnostics are carried out for the gas status variable to be measured by a comparison of a modelled signal and a measured signal, and wherein the measured signal is an actual value of an output signal of the gas sensor, and the modelled signal is a model value, **characterized in that** in a steady-state operating mode of the internal combustion engine (10), the output signal of the gas sensor is filtered with a high-pass filter (23) and relatively high frequency signal components are evaluated by means of a comparison with the correspondingly conditioned model value.

2. Method according to Claim 1, **characterized in that** a rate of change of the gas status variables to be measured is determined, and a steady-state operating mode of the internal combustion engine (10) is detected therefrom.

3. Method according to Claim 1 or 2, **characterized in that** the energy or the power of the relatively high frequency signal components of the gas sensor and of correspondingly high-pass filtered output signals from a model (31) of the gas sensor are compared with threshold values for the energy or the power, and on the basis of the comparison the presence of electrical faults in the gas sensor or the occurrence of vibrations is detected.

4. Method according to Claim 3, **characterized in that** when an upper threshold value for the energy or the power of the relatively high frequency signal components of the gas sensor is exceeded and at the same time a lower threshold value for the energy or the power of the relatively high frequency signal components of the modelled signal which is determined from the model (31) is undershot, an electrically vibrating gas sensor is detected.

5. Method according to one of Claims 1 to 4, **characterized in that** a model time constant $T_M$, stored in the model (31), corresponds to that of a nominal gas sensor and/or this model time constant $T_M$ and the threshold values are adapted as a function of the gas status variables.

6. Method according to one of Claims 1 to 5, **characterized in that** the integration of the relatively high frequency signal components of the sensor signal and of the modelled signal is started in an eventcontrolled fashion or when the steady-state operating mode of the internal combustion engine (10) is detected, and is ended in a time-controlled or eventcontrolled fashion.

7. Method according to Claim 6, **characterized in that** in the event of an event control the threshold values are adapted as a function of a current integration period $T_{current}$, which deviates from a typical integration period $T_{norm}$.

8. Method according to one of Claims 1 to 6, **characterized in that** an average signal power of the relatively high frequency signal components of the sensor signal and of the modelled signal are evaluated, and comparison is carried out with an applicable power threshold value.

9. Method according to one of Claims 1 to 8, **characterized in that** during the high-pass filtering the signal of the gas sensor and/or of the modelled signal are/is additionally filtered with filter units (32, 33) or filter functions which have, in their characteristic curve profile, insensitivity zones or dead zones in the region of small input variables, wherein the latter can additionally have discontinuities in their characteristic curve profile.

10. Method according to Claim 9, **characterized in that** the characteristic curve profiles of these filter units (32, 33) or filter functions are combined with the functionality of the square of the signals, or the absolute value of the high-pass signal output is used, or the formation of absolute values is combined with the insensitivity zone or dead zone in a characteristic curve of the filter units (32, 33).

11. Method according to one of Claims 1 to 10, **characterized in that**, as gas sensors, gas pressure sensors, gas temperature sensors, gas mass flow sensors or gas concentration sensors in the form of exhaust gas probes (15) are used in the exhaust gas duct (18) of the internal combustion engine (10) as part of an exhaust gas monitoring and reducing system, or in an air intake line (11) of the internal combustion engine (10).

12. Method according to one of Claims 1 to 11, **characterized in that** exhaust gas probes (15) in the form of broadband lambda sensors or $NO_x$ sensors with which an oxygen content in a gas mixture can be determined are used as gas sensors.

13. Method according to one of Claims 1 to 12, **characterized in that** the monitoring method according to Claims 1 to 12 is combined with methods for dynamic diagnostics of gas sensors, in which method, for the purpose of dynamic diagnostics, relatively high frequency signal components are evaluated when there is a change in the gas status variable to be measured.

14. Device for monitoring gas sensors in the exhaust gas duct of an internal combustion engine (10) as part of an exhaust gas monitoring and reducing system or in an air intake line of the internal combustion engine (10), wherein the gas sensors have a low-pass behaviour depending on the geometry, measuring principle, ageing or soiling, wherein diagnostics are carried out in a monitoring unit for the gas status variable to be measured on the basis of a comparison of a modelled signal and a measured signal, and wherein the measured signal is an actual value of an output signal of the gas sensor, and the modelled signal is a model value, **characterized in that** the monitoring unit has high-pass filters (23) for evaluating relatively high frequency signal components as well as at least one model (31) for the gas sensor and calculation units and additional filter units (32, 33) or filter functions for carrying out the monitoring according to Claims 1 to 13.

**Revendications**

1. Procédé de surveillance de capteurs de gaz d'un moteur à combustion interne (10), dans lequel les capteurs de gaz présentent un comportement passe-bas en fonction de la géométrie, du principe de mesure, du vieillissement ou de la salissure, dans lequel un diagnostic est effectué sur les grandeurs d'état des gaz à mesurer en comparant un signal modélisé et un signal mesuré et dans lequel le signal mesuré est une valeur observée d'un signal de sortie du capteur de gaz et le signal modélisé est une valeur de modèle, **caractérisé en ce que**, lors d'un fonctionnement en régime stationnaire du moteur à combustion interne (10), le signal de sortie du capteur de gaz est filtré au moyen d'un filtre passe-haut (23) et des parties de signal à haute fréquence sont évaluées par comparaison à la valeur de modèle traitée correspondante.

2. Procédé selon la revendication 1, **caractérisé en ce qu'**une vitesse de variation des grandeurs d'état des gaz à mesurer est obtenue et **en ce qu'**un fonctionnement en régime stationnaire du moteur à combustion interne (10) est détecté à partir de celle-ci.

3. Procédé selon la revendication 1 ou 2, **caractérisé en ce que** l'énergie ou la puissance des parties de signal à haute fréquence du capteur de gaz et de signaux de sortie correspondants filtrés par filtrage passe-haut provenant d'un modèle (31) du capteur de gaz est comparée à des valeurs de seuil pour l'énergie ou la puissance et **en ce qu'**il est déterminé sur la base de la comparaison si des défauts électriques du capteur de gaz sont présents et si des oscillations se produisent.

4. Procédé selon la revendication 3, **caractérisé en ce que**, lors du dépassement d'une valeur de seuil supérieure pour l'énergie ou la puissance des parties de signal à haute fréquence du capteur de gaz et lors du l'abaissement simultané en dessous d'une valeur de seuil inférieure pour l'énergie ou la puissance des parties de signal à haute fréquence du signal modélisé déterminé à partir du modèle (31), il est déterminé que le capteur de gaz oscille électriquement.

5. Procédé selon l'une quelconque des revendications 1 à 4, **caractérisé en ce qu'**une constante de temps de modèle $T_M$ utilisée dans le modèle (31) correspond à celle d'un capteur de gaz nominal et/ou **en ce que** celle-ci ainsi que les valeurs de seuil sont adaptées en fonction des grandeurs d'état des gaz.

6. Procédé selon l'une quelconque des revendications 1 à 5, **caractérisé en ce que** l'intégration des parties de signal à haute fréquence du signal de capteur et du signal modélisé est interrompue de manière commandée par événement ou est lancée lorsqu'il est détecté que le moteur à combustion interne (10) fonctionne en régime stationnaire et est interrompue d'une manière commandée dans le temps ou par événement.

7. Procédé selon la revendication 6, **caractérisé en ce que**, dans le cas d'une commande par événement, les valeurs de seuil sont adaptées en fonction d'un temps d'intégration courant $T_{courant}$ qui s'écarte d'un temps d'intégration typique $T_{norm}$.

8. Procédé selon l'une quelconque des revendications 1 à 6, **caractérisé en ce qu'**une puissance de signal moyenne de la partie de signal à haute fréquence du capteur de signal et du signal modélisé est évaluée et **en ce que** la comparaison est effectuée avec une valeur de seuil de puissance applicable.

9. Procédé selon l'une quelconque des revendications 1 à 8, **caractérisé en ce que**, lors du filtrage passe-haut, le signal du capteur de gaz et/ou le signal modélisé sont en outre filtrés par des unités de filtrage (32, 33) ou des fonctions de filtrage qui présentent dans leur courbe caractéristique des zones d'insensibilité ou des zones mortes dans une région où les grandeurs d'entrée sont plus faibles, celles-ci pouvant en outre présenter des sauts dans leur courbe caractéristique.

**10.** Procédé selon la revendication 9, **caractérisé en ce que** la courbe caractéristique desdites unités de filtrage (32, 33) ou desdites fonctions de filtrage sont combinées avec la fonctionnalité de l'élévation au carré des signaux ou **en ce que** la valeur de la sortie de signal passe-haut est utilisée ou également **en ce que** la détermination est combinée avec la zone d'insensibilité ou zone morte en une ligne caractéristique des unités de filtrage (32, 33).

**11.** Procédé selon l'une quelconque des revendications 1 à 10, **caractérisé en ce que** l'on utilise en tant que capteurs de gaz des capteurs de pression de gaz, des capteurs de température de gaz, des capteurs de débit massique de gaz ou des capteurs de concentration en gaz sous la forme de sondes de gaz d'échappement (15) dans le conduit de gaz d'échappement (18) du moteur à combustion interne (10), en tant que partie d'un système de surveillance et de réduction ou dans un conduit d'admission d'air (11) du moteur à combustion interne (10).

**12.** Procédé selon l'une quelconque des revendications 1 à 11, **caractérisé en ce que** l'on utilise en tant que capteurs de gaz des sondes de gaz d'échappement (15) sous la forme de sondes lambda à large bande ou de capteurs de $NO_x$ au moyen desquels il est possible de déterminer une teneur en oxygène d'un mélange de gaz.

**13.** Procédé selon l'une quelconque des revendications 1 à 12, **caractérisé en ce que** le procédé de surveillance selon les revendications 1 à 12 est combiné à un procédé de diagnostic dynamique des capteurs de gaz au moyen duquel une évaluation de parties de signal à haute fréquence est effectuée à des fins de diagnostic dynamique lors d'une modification des grandeurs d'état des gaz à mesurer.

**14.** Dispositif de surveillance de capteurs de gaz dans un conduit de gaz d'échappement d'un moteur à combustion interne (10) en tant que partie d'un système de surveillance et de réduction ou dans un conduit d'admission d'air du moteur à combustion interne (10), dans lequel les capteurs de gaz présentent un comportement passe-bas en fonction de la géométrie, du principe de mesure, du vieillissement ou de la salissure, dans lequel un diagnostic est effectué sur les grandeurs d'état des gaz à mesurer sur la basé d'une comparaison d'un signal modélisé et d'un signal mesuré dans une unité de surveillance et dans lequel le signal mesuré est une valeur observée d'un signal de sortie du capteur de gaz et le signal modélisé est une valeur de modèle, **caractérisé en ce que** l'unité de surveillance comporte un filtre passe-haut (23) destiné à évaluer des parties de signal à haute fréquence ainsi qu'au moins un modèle (31) du capteur de gaz et des unités de calcul et des unités de filtrage (32, 33) ou des fonctions de filtrage supplémentaires destinées à mettre en oeuvre la surveillance selon des revendications 1 à 13.

**Fig. 1**

Fig. 2

**23**

**32**

**25**

**22.1**

**25.1**

**24**

**Fig. 3a**

**23**

**33**

**25**

**22.1**

**25.1**

**24**

**Fig. 3b**

**23**

**32**

**25**

**22.1**

**25.1**

**Fig. 4a**

Fig. 4b

Fig. 5a

Fig. 5b

Fig. 6

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102008001121 A1
- DE 102008040737 A1
- DE 102008001569 A1